# EUROPÄISCHE PATENTSCHRIFT

(11) **EP 0 314 896 B1**
(45) Veröffentlichungstag und Bekanntmachung des Hinweises auf die Patenterteilung: **20.04.1994**
(21) Anmeldenummer: 88114551.0
(22) Anmeldetag: 07.09.1988
(51) Int. Cl.: A61B 17/22

(54) **Vorrichtung zur Thromboembolectomie**
Thromboembolectomy device
Dispositif de thromboembolectomie

(30) Priorität: 31.10.1987 DE 8714529 U
(43) Veröffentlichungstag der Anmeldung: 10.05.1989
(73) Patentinhaber: ANGIOMED AG, 76227 Karlsruhe (DE)
(72) Erfinder: Schnepp-Pesch, Wolfram, D-7505 Ettlingen (DE); Lindenberg, Josef, D-7500 Karlsruhe 1 (DE)
(74) Vertreter: Dipl.-Ing. Heiner Lichti Dipl.-Phys. Dr.rer.nat. Jost Lempert Dipl.-Ing. Hartmut Lasch

(56) Entgegenhaltungen:
- EP-A- 0 177 782
- US-A- 3 169 528
- US-A- 3 499 435
- US-A- 4 601 713
- US-A- 4 755 176

## Beschreibung

Die Erfindung betrifft eine Vorrichtung zur Thromboembolectomie unter Anlösen durch Streptokinase oder dergleichen.

Es sind aus der Praxis gattungsgemäße Vorrichtungen zur Thromboembolectomie bekannt, die einen Führungsdraht und einen über diesen führbaren Katheter mit einem distal und proximal offenen Lumen, das proximal an eine Infusions- und/oder Saugspritze anschließbar ist, aufweisen. Bei dieser bekannten Vorrichtung wird derart vorgegangen, daß zunächst der Führungsdraht durch das Gewebe des Patienten in eine Vene und entlang dieser bis zum zu entfernenden Thrombus gelegt wird. Anschließend werden der Katheter über den Führungsdraht bis zum Thrombus eingeführt, der Führungsdraht entfernt, gegebenenfalls durch den Katheter ein den Thrombus an- bzw. auflösendes Mittel, wie Streptokinase, gespritzt und anschließend Thrombusteile angesaugt. Es konnte dabei vorkommen, daß die Thrombusteile stecken blieben und die gesamten, in die Vene eingeführten Teile mit einem Thrombusteil aus dem Körper entfernt werden mußten. Anschließend mußte die gesamte Prozedur mit Punktion und gesteuerter, röntgenbeobachteter Einführung des Führungsdrahtes wiederholt werden. Dies war für den Patienten belastend.

Aus der Praxis ist ein nicht gattungsgemäßer Katheter zur Zerstörung von Blutgefäßblockierungen mittels Laserenergie bekannt, der mittels seines Lumens über einen Führungsdraht eingeführt und durch dessen Lumen Kühl- und Waschflüssigkeit injiziert werden können, wobei weiterhin in der Wandung eine optische Faser eingebettet ist, durch die die Laserenergie zum distalen Ende des Katheters geführt wird. Es sind weiterhin doppellumige Ballonkatheter bekannt, bei denen ein zweites Lumen in der Seitenwand des Katheters radial unter einer um den Katheter befestigten elatischen, als Ballon aufblasbaren Folie mündet.

Die EP-A-177 782 zeigt eine Vorrichtung zur Thromboembolectomie mit einer durch einen Katheter geführten, von ihrem rückwärtigen Ende her angetriebenen Antriebswelle, die an ihrem vorderen, aus dem Katheter herausragenden Ende einen Schneidkopf aufweist, mit dem der Thrombus mechanisch aufgebrochen wird, wobei das abgeschnittene Material durch den Katheter abgesaugt werden kann.

Der Erfindung liegt die Aufgabe zugrunde, eine Vorrichtung zur Thromboembolectomie mittels Anlösens und Absaugens von Thrombusmaterial zu schaffen, mittels der die Belastung des Patienten reduziert und eine größere Variationsmöglichkeit hinsichtlich der Durchführung der Thrombusentfernung ermöglicht wird.

Erfindungsgemäß wird die genannte Aufgabe gelöst durch ein Katheterbesteck mit einer doppellumigen Schleuse mit zwei sich über den größten Teil der Länge der Schleuse parallel erstreckenden, beidseitig offenen Lumen, die distal parallel zueinander, proximal unter einer endlichen Winkelausrichtung münden, wobei das erste Lumen geradezu in den gesamten Querschnittsbereich der Schleuse einnimmt, während das zweite Lumen wesentlich enger ausgeführt und in der Wandung des ersten Lumens ausgebildet ist, und mit einem an das zweite Lumen angepaßten Sicherheits-Wechseldraht.

Durch die erfindungsgemäße Vorrichtung ist es möglich, die gesamte Schleuse, einschließlich gegebenenfalls eines in ihr eingeführten Katheters, bei ein- bzw. angesaugten Thrombusteilen aus dem Körper des Patienten zu entfernen, dabei aber den Sicherheits-Wechseldraht liegen zu lassen und anschließend in bequemer Weise, ohne daß eine erneute vollständige Punktion und unter Röntgenbetrachtung gesteuerte Einführung erforderlich ist, die Schleuse bzw. eine neue Schleuse wieder zum Thrombus einzuführen. In bevorzugter Ausgestaltung sieht die Erfindung einen an den Querschnitt des ersten Lumens angepaßten ersten Katheter vor. In diesem Falle kann die Wandung der Schleuse äußerst dünn ausgeführt werden, da sie keine Eigensteifigkeit mehr aufweisen muß, sondern diese gegebenenfalls durch den Katheter gewährleistet ist. Eine Weiterbildung der erfindungsgemäßen Vorrichtung ist gekennzeichnet durch einen derart an Schleuse und/oder ersten Katheter angepaßten Führungsdraht, daß jene über ihn einführbar sind und weiterhin durch eine an den Führungsdraht mit ihrem Lumen angepaßte Punktionskanüle. Durch diese besteck- oder setartige Ausgestaltung der erfindungsgemäßen Vorrichtung wird erreicht, daß der Operateur ohne weiteres geeignete und angepaßte weitere Teile, Punktionskanüle und Führungsdraht zur Verfügung hat, die mit den sonstigen Teilen der erfindungsgemäßen Vorrichtung, insbesondere dem erfindungsgemäß ausgebildeten Katheterbesteck, abgestimmt sind und optimal eingesetzt werden können. Hierzu wird weiterhin mindestens eine Spritze zum Injizieren einer thrombuslösenden Flüssigkeit, wie Streptokinase, und/oder Absaugen von Thrombusmaterial vorgesehen, wobei die Spritzen derart mit Adaptern versehen sind, daß sie an entsprechende Adapter der Schleuse und/oder der Katheter problemlos und ohne weiteres angesetzt werden können, insbesondere durch eine bajonettartige Ausgestaltung der Adapter. Soweit eine Injektionsspritze vorgesehen ist, so kann diese sogleich mit einem den Thrombus lösenden Mittel, wie Streptokinase, gefüllt sein, so daß sie sofort einsatzfähig ist und Streptokinase nicht zunächst aufgezogen werden muß. In disem Falle ist in bevorzugter Ausgestaltung vorgesehen, daß der Adapter der Spritze ein geschlossenes Ende aufweist, welches abgeschnitten werden kann, so daß über die dadurch geschaffene Öffnung die Flüssigkeit injiziert werden kann.

In weiterer bevorzugter Ausgestaltung ist vorgesehen, daß der erste Katheter eine eng ausgezogene, sich verjüngende distale Spitze aufweist, wobei weiterhin ein zweiter Katheter mit einem seinem Lumenquerschnitt entsprechenden offenen distalen Ende ausgebildet ist, dessen Querschnitt dem Lumenquerschnitt des Katheters über seine gesamte Länge hin entspricht. Der Katheter mit sich verjüngendem distalen Ende erleichtert die Einführung des Katheterbestecks bis zum Thrombus, während ein Katheter mit einem breiten offenen distalen Ende ermöglicht, größere Thrombusteile an und durch den Katheter hindurchzusaugen, als dies bei einem Katheter mit verjüngtem Ende der Fall ist. Damit bei vorgegebenem Außendurchmesser der Schleuse im Bereich von 3 2/3 bis 4 mm (11 bis 12 Charrière (Ch)) ein möglichst großer Innendurchmesser für Katheter und durchzusaugende Thrombusstücke zur Verfügung steht, so daß möglichst große Thrombusstücke hindurchgesaugt werden können, sollte neben der Wandstärke der Schleuse, die vorzugsweise im Bereich von 0,2 mm oder insbesondere unter diesem Wert liegt, auch der Innendurchmesser des zweiten Lumens der Schleuse und damit der Querschnitt des Sicherheits-Wechseldrahtes möglichst klein sein und liegt insbesondere unter 0,5 mm und äußerst vorteilhaft ebenfalls unter 0,2 mm, damit in der Größenordnung der dünnen Wandung der Schleuse. Damit steht bei einer Schleuse mit dem genannten Außendurchmesser ein Innendurchmesser des großen Lumens im Bereich von 2 2/3 bis 3 1/3 mm (8 bis 10 Charrière) bei vertretbaren Außenabmessungen zur Verfügung.

Weitere Vorteile und Merkmale der Erfindung ergeben sich aus den Ansprüchen und aus der nachfolgenden Beschreibung, in der ein Ausführungsbeispiel der erfindungsgemäßen Vorrichtung unter Bezugnahme auf die Zeichnung im einzelnen erläutert ist. Dabei zeigt:
- Figur 1: einen Schnitt durch ein Katheterbesteck nach der Erfindung; und
- Figur 2: die erfindungsgemäße Vorrichtung in Seitenansicht anhand verschiedener Einsatzschritte entsprechend den Figuren 2a bis 2e.

Die erfindungsgemäße Vorrichtung 1 weist ein Katheterbesteck 2 mit einer Schleuse 3, mit einem ersten Lumen 4 und einem zweiten Lumen 6 auf. Das erste Lumen 4 hat einen Querschnitt, der nahezu den gesamten Querschnitt der Schleuse 3 ausfüllt und beispielsweise im Bereich von 3,5 mm bei einem Außendurchmesser von ca. 3,8 mm liegt, während das zweite Lumen 6 in der Wandung 7 der Schleuse 3 ausgebildet ist und einen wesentlich geringeren Querschnitt mit einem Durchmesser in der Größenordnung von 0,6 mm hat. Die Wand 7 der Schleuse ist, wie sich aus Vorstehendem ergibt, äußerst dünn und daher hoch flexibel. Sie weist geringe Wandstärken im Bereich von 0,1 bis 0,2 mm auf. In der Schleuse 3 ist ein Katheter 8 eingeführt, so daß die gesamte Einheit mit dem Lumen 9 des Katheters 8 über einen Führungsdraht eingeführt werden konnte. In dem zweiten Lumen der Schleuse 3 befindet sich ein Sicherheits-Wechseldraht 11. Durch das Lumen 9 des Katheters 8 kann nun mittels einer Spritze Streptokinase hindurch injiziert werden. Weiterhin können durch das Lumen 9 des Katheters mittels einer Vakuumspritze Thrombusteile angesaugt werden, so daß diese mit dem Katheter durch das erste Lumen 4 der Schleuse 3 hindurchgesogen oder aber gemeinsam mit der Schleuse 3 herausgezogen werden, wobei der Sicherheits-Wechseldraht 11 liegen bleibt. Gegebenenfalls kann auch bei entferntem Katheter 8 ein Ansaugen direkt durch die Schleuse 3 erfolgen, wenn diese eine hinreichende Eigenstabilität aufweist, wobei hier in der Regel zwischen möglichst dünner Wandungsstärke oder hinreichender Eigenstabilität auch gegen Saugdruck aufweisende Wandungsstabilität zu entscheiden ist.

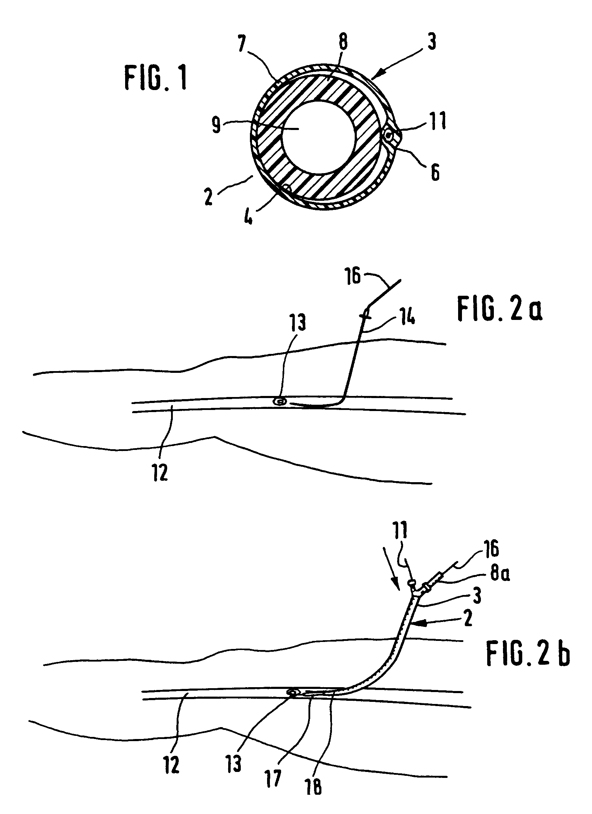

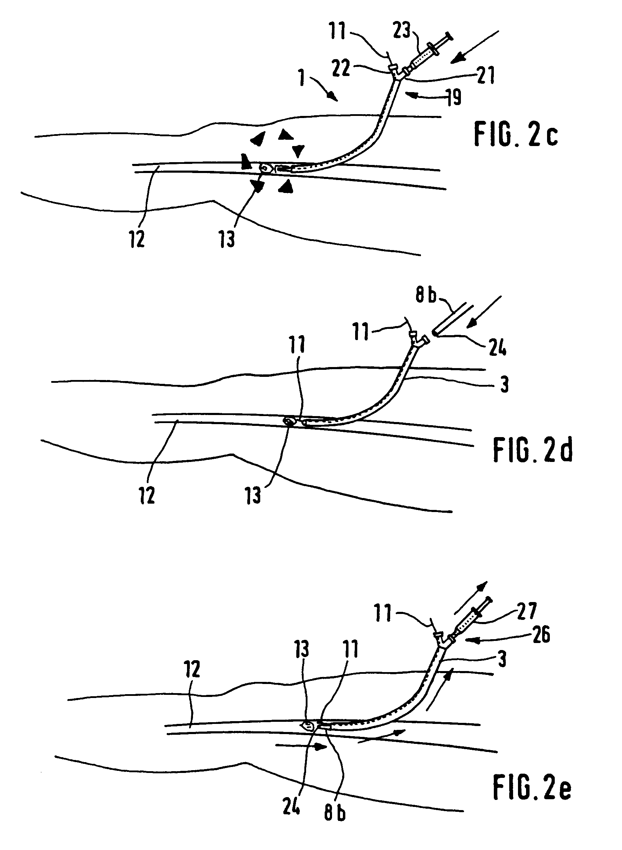

In der Figur 2 ist ein beispielhafter Ablauf des Einsatzes der erfindungsgemäßen Vorrichtung zur Thromboembolectomie mittels Streptokinase dargestellt, wobei auch weitere Elemente der erfindungsgemäßen Vorrichtung beschrieben werden.

Zunächst wird die Vene 12 eines Patienten nahe eines in ihr befindlichen Thrombus 13 mittels einer Punktionskanüle 14, wie einer Seldinger-Punktionskanüle, punktiert. Anschließend wird ein Führungsdraht 16 durch die Punktionskanüle 14 bis nahe zum Thrombus 13 eingeführt. In einem nächsten Schritt wird die Punktionskanüle 14 über den Führungsdraht 16 abgezogen, der liegen bleibt. Anschließend wird das Katheterbesteck 2 mit insbesondere Schleuse 3 und vorzugsweise in deren größerem Lumen 4 befindlichen Katheter 8a sowie im zweiten Lumen mit geringem Querschnitt befindlichen Sicherheits-Wechseldraht 11 über den Führungsdraht 16 bis in die Vene 12 und ebenfalls nahe zum Thrombus 13 eingeführt. Gegebenenfalls kann der Sicherheits-Wechseldraht 11 auch erst nach Einführen der Schleuse 3 durch deren zweites Lumen 6 eingeschoben werden.

Der Katheter 8a ist vorzugsweise ein sogenannter Van-Andel-Katheter, der ein zugespitztes Ende 17 und eine größere Länge als die Schleuse 3 aufweist, so daß er mit seinem distalen Ende 17 über das distale Ende 18 der Schleuse hinausragt. Durch die Ausgestaltung des Van-Andel-Katheters 8a wird die Einführung des Katheterbestecks erleichtert.

Anschließend wird der Führungsdraht 16 aus dem Lumen 9 des Katheters 8a entfernt.

Die Schleuse 3 und/oder der Katheter 8a weisen an ihren proximalen Enden im Bereich 19 Adapter 21, 22, wie Luer-Adapter, auf. Hierzu sind die proximalen Mündungen der Lumen 4, 6 der Schleuse 3, die ansonsten über die gesamte Länge der Schleuse 3 parallel zueinander verlaufen, unter einem endlichen Winkel jeweils voneinander weg gerichtet, so daß an beiden Lumen-Mündungen frei und ungestört hantiert werden kann. Der Winkel zwischen den proximalen Lumen-Mündungen liegt über 30°, vorzugsweise im Bereich von 45 und 90°, wobei beide Mündungen zur Achse der Rest-Schleuse etwa den gleichen Winkel einschließen. An einen Adapter 21 kann nun eine mit Streptokinase gefüllte Injektionsspritze 23 angesetzt werden, durch welche Streptokinase in die Vene 12 bis in den Bereich des Thrombus 13 eingespritzt werden kann. Die Streptokinase bewirkt in bekannter Weise ein An- und teilweises Auflösen des Thrombus, so daß dieser, der zunächst in der Vene 12 festsitzt, gelöst wird bzw. Teile von ihm gelöst werden.

In äußerst bevorzugter Weise wird der Van-Andel-Katheter 8a aus der Schleuse 3 herausgezogen und ein weiterer Katheter 8b durch die Schleuse bis zum Thrombus 13 eingeführt. Der Katheter 8b unterscheidet sich dabei vom Van-Andel-Katheter 8a durch eine größere distale Öffnung 24, durch die größere Teile des Thrombus 13 ein- bzw. an der Öffnung 24 festgesaugt werden können.

Am proximalen Ende 26 des Saugkatheters 8b wird an einem ebenfalls dort vorhandenen Adapter eine Saugspritze 27 angesetzt, mit der Thrombusteile durch den Katheter 8b gesaugt bzw. in oder aber an der Mündung 24 des Katheters 8b festgesaugt werden können.

Soweit die Thrombusteile geringere Außenabmessungen als der Innendurchmesser der Schleuse 3 aufweisen, können sie, auch wenn sie nur an der Mündung 24 des Katheters 8b sitzen, mit diesem durch die Schleuse 3 hindurch aus dem Körper des Patienten herausgezogen und entfernt werden. Da man zur Abkürzung der Entfernung des Thrombus und zur Reduzierung der gesamten Belastung des Patienten versucht, möglichst große Thrombusteile zu entfernen, kann es passieren, daß ein solcher im Bereich der distalen Mündung der Schleuse 3 oder in dieser hängen bleibt, so daß die gesamte Schleuse 3 aus dem Körper des Patienten zu entfernen ist. Sie wird dann über den Sicherheits-Wechseldraht 11 mit dem Thrombusteil herausgezogen, wobei der Sicherheitsdraht 11 im Körper und mit seinem distalen Ende nahe des Thrombus 13 liegen bleibt. Anschließend kann über und entlang des Sicherheitsfadens 11 das gleiche oder ein neues Katheterbesteck zum Thrombus eingeführt werden, wie dies weiter oben beschrieben wurde und das Entfernen des Thrombus durch Anlösen mittels Streptokinase und Absaugen von Thrombusteilen fortgesetzt werden. Die abwechselnde Infusion/Aspiration wird so lange fortgeführt, bis der Thrombus vollständig entfernt ist.

Die erfindungsgemäße Vorrichtung bietet eine große Variabilität hinsichtlich der Thrombusentfernung. So kann, wie gesagt, soweit die Schleuse eine geeignete Eigensteifigkeit aufweist, die Aspiration bei großen Thrombusfragmenten auch direkt durch die Schleuse vorgenommen werden. Durch den im zweiten Lumen der Schleuse 3 liegenden Sicherheits-Wechseldraht 11 bis über die gesamte Dauer des Eingriffs ist eine Führungsschiene gegeben, die auch in axialer Richtung eine Stabilisierung bewirkt. Darüber hinaus kann gegebenenfalls zum Absaugen auch der Van-Andel-Katheter 8a oder zum Einführen und Einspritzen ein Katheter 8b mit größerer distaler Öffnung in geeigneten Fällen eingesetzt werden.

Weiterhin kann durch das zweite Lumen mit geringerem Querschnitt Streptokinase injiziert werden, während gleichzeitig über das erste Lumen Thrombusteile aspiriert werden.

## Patentansprüche

1. Vorrichtung zur Thromboembolectomie unter Anlösen durch Streptokinase oder dergleichen, mit einem Katheterbesteck (2) mit einer doppellumigen Schleuse (3) mit zwei sich über den größten Teil der Länge der Schleuse (3) parallel erstreckenden, beidseitig offenen Lumen (4, 6), die distal parallel zueinander, proximal unter einer endlichen Winkelausrichtung münden, wobei das erste Lumen (4) nahezu den gesamten Querschnittsbereich der Schleuse (3) einnimmt, während das zweite Lumen (6) wesentlich enger ausgeführt und in einen erweiterten Bereich der Wandung (7) des ersten Lumens ausgebildet ist, und mit einem an das zweite Lumen (6) angepaßten Sicherheits-Wechseldraht (11).

2. Vorrichtung nach Anspruch 1, gekennzeichnet durch mindestens einen an den Querschnitt des ersten Lumens (4) angepaßten ersten Katheter (8, 8a).

3. Vorrichtung nach Anspruch 1 oder 2, gekennzeichnet durch einen derart an Schleuse (3) und/oder ersten Katheter (8, 8a) angepaßten Führungsdraht (16), daß jene über ihn einführbar sind.

4. Vorrichtung nach einem der vorangehenden Ansprüche, gekennzeichnet durch eine an den Führungsdraht (16) mit ihrem Lumen angepaßte Punktionskanüle (14).

5. Vorrichtung nach einem der vorangehenden Ansprüche, gekennzeichnet durch mindestens eine Spritze (23,27) zum Injizieren einer thrombuslösenden Flüssigkeit, wie Streptokinase, und/oder Absaugen von Thrombusmaterial.

6. Vorrichtung nach einem der Ansprüche 2 bis 5, dadurch gekennzeichnet, daß der ersten Katheter (8a) eine eng ausgezogene, sich verjüngende distale Spitze aufweist.

7. Vorrichtung nach einem der Ansprüche 2 bis 6, dadurch gekennzeichnet, daß ein zweiter Katheter (8b) mit einem seinem Lumenquerschnitt entsprechenden offenen distalen Ende ausgebildet ist, dessen Querschnitt dem Lumenquerschnitt des Katheters (8b) über seine gesamte Länge hin entspricht.

8. Vorrichtung nach einem der vorangehenden Ansprüche, dadurch gekennzeichnet, daß die Schleuse eine Wandstärke von unter 0,2 mm aufweist.

9. Vorrichtung nach einem der vorangehenden Ansprüche, dadurch gekennzeichnet, daß der Innendurchmesser des Lumens der Schleuse im Bereich von 2 2/3 bis 4 mm (8 bis 12 Charrière (Ch)) liegt.

10. Vorrichtung nach einem der vorangehenden Ansprüche, dadurch gekennzeichnet, daß der Außendurchmesser der Schleuse (3) im Bereich von 3 2/3 bis 4 2/3 mm (11 bis 14 Ch) liegt.

11. Vorrichtung nach einem der vorangehenden Ansprüche, dadurch gekennzeichnet, daß der Innendurchmesser des zweiten Lumens (6) der Schleuse (3) kleiner 0,7 mm, insbesondere kleiner als 0,4 mm ist.

## Claims

1. Instrument for thrombectomy accompanied by partial dissolving by streptokinase or the like, with a catheter set (2) having a double-opening lock (3) with two parallel openings (4,6), open on both sides, extending over most of the length of the lock (3), which are distal-parallel to one another and issue proximally under a finite angular orientation, the first opening (4) taking up almost the entire cross-sectional area of the lock (3), whereas the second opening (6) is much narrower and is formed in a widened area of the wall (7) of the first opening and having a safety interchangeable wire (11) adapted to the second opening (6).

2. Instrument according to claim 1, characterized by at least one first catheter (8,8a) adapted to the cross-section of the first opening (4).

3. Instrument according to claim 1 or 2, characterized by a guide wire (16) adapted to the lock (3) and/or the first catheter (8,8a) in such a way that they can be inserted via the same.

4. Instrument according to one of the preceding claims, characterized by a puncture cannula (14) adapted by its opening to the guide wire (16).

5. Instrument according to one of the preceding claims, characterized by at least one syringe (23,27) for injecting a thrombus-dissolving liquid, such as streptokinase, and/or for the suction of thrombus material.

6. Instrument according to one of the claims 2 to 5, characterized in that the first catheter (8a) has a narrow, stretched, tapering, distal tip.

7. Instrument according to one of the claims 2 to 6, characterized in that a second catheter (8b) with an open distal end corresponding to its opening cross-section is formed and whose cross-section corresponds to the opening cross-section of the catheter (8b) over its entire length.

8. Instrument according to one of the preceding claims, characterized in that the lock has a wall thickness of below 0.2 mm.

9. Instrument according to one of the preceding claims, characterized in that the internal diameter of the opening of the lock is in the range 2 2/3 to 4 mm (8 to 12 Charrière (Ch)).

10. Instrument according to one of the preceding claims, characterized in that the external diameter of the lock (3) is in the range 3 2/3 to 4 2/3 mm (11 to 14 Ch).

11. Instrument according to one of the preceding claims, characterized in that the internal diameter of the second opening (6) of the lock (3) is smaller than 0.7 mm and in particular smaller than 0.4 mm.

## Revendications

1. Dispositif de thromboembolectomie sous l'action dissolvante de la streptokinase ou analogue, comprenant un jeu de cathéter (2) à cathéter double lumières (3), dont les deux lumières (4,6) latéralement juxtaposées s'étendent parallèlement sur la majeure partie de la longueur du cathéter double lumières (3) et débouchent du côté distal parallèles l'une à l'autre, du côté proximal sous une pièce d'angle terminale, la première lumière (4) occupant la quasi totalité de la section transversale du cathéter double lumières (3), alors que la deuxième lumière (6) est sensiblement plus étroite et conformée dans un espace élargi de la paroi (7) de la première lumière, dispositif comprenant un fil de rechange de sécurité (11) adapté à la deuxième lumière (6).

2. Dispositif selon la revendication 1, caractérisé par au moins un premier cathéter (8,8a) adapté à la section de la première lumière (4).

3. Dispositif selon l'une des revendications 1 ou 2, caractérisé par un fil de guidage (16) adapté au cathéter double lumières (3) et/ou au premier cathéter (8,8a) de manière que chacun de ceux-ci puisse être introduit au-dessus de lui.

4. Dispositif selon l'une quelconque des revendications précédentes, caractérisé par une canule de ponction (14) adaptée par sa lumière au fil de guidage (16).

5. Dispositif selon l'une quelconque des revendications précédentes, caractérisé par au moins une seringue (23,27) pour l'injection d'un liquide dissolvant le thrombus tel que la streptokinase et/ou pour l'évacuation par succion des particules de thrombus.

6. Dispositif selon l'une quelconque des revendications 2 à 5, caractérisé en ce que le premier cathéter (8a) présente une pointe distale étroitement étirée allant en se rétrécissant.

7. Dispositif selon l'une quelconque des revendications 2 à 6, caractérisé en ce qu'un deuxième cathéter (8b) comporte une extrémité distale ouverte correspondant à la section de sa lumière, dont la section correspond sur toute sa longueur à la section de la lumière du cathéter (8b).

8. Dispositif selon l'une quelconque des revendications précédentes, caractérisé en ce que le cathéter double lumières (3) présente une épaisseur de paroi inférieure à 0,2 mm.

9. Dispositif selon l'une quelconque des revendications précédentes, caractérisé en ce que le diamètre intérieur de la lumière du cathéter double lumières (3) se situe entre 2 2/3 et 4 mm (8 à 12 Charrière (Ch)).

10. Dispositif selon l'une quelconque des revendications précédentes, caractérisé en ce que le diamètre extérieur du cathéter double lumières (3) se situe entre 3 2/3 et 4 2/3 mm (11 à 14 Ch).

11. Dispositif selon l'une quelconque des revendications précédentes, caractérisé en ce que le diamètre intérieur de la deuxième lumière (6) du cathéter double lumières (3) est inférieur à 0,7 mm, en particulier inférieur à 0,4 mm.
